(19) Europäisches Patentamt
European Patent Office
Office européen des brevets

(11) EP 0 507 323 B1

(12) EUROPEAN PATENT SPECIFICATION

(45) Date of publication and mention of the grant of the patent:
**29.01.1997 Bulletin 1997/05**

(51) Int Cl.6: **C12P 19/02**, C12P 19/12, C12P 19/44, C12P 7/64

(21) Application number: **92105783.2**

(22) Date of filing: **03.04.1992**

# (54) Process for preparing fatty acid esters of saccharides

Verfahren zur Herstellung von Fettsäuresaccharidestern

Procédé de préparation d'esters d'acides gras avec des saccharides

(84) Designated Contracting States:
**DE DK ES FR GB IT NL**

(30) Priority: **05.04.1991 JP 100484/91**
**02.10.1991 JP 282088/91**
**02.10.1991 JP 282089/91**

(43) Date of publication of application:
**07.10.1992 Bulletin 1992/41**

(73) Proprietor: **LION CORPORATION**
**Sumida-ku Tokyo (JP)**

(72) Inventors:
- **Andoh, Hideo**
  **Tokyo (JP)**
- **Tamiya, Masakatu**
  **Yokohama-shi, Kanagawa-ken (JP)**
- **Iwasaki, Ryozo**
  **Chiba-shi, Chiba-ken (JP)**

(74) Representative: **VOSSIUS & PARTNER**
**Postfach 86 07 67**
**81634 München (DE)**

(56) References cited:
**WO-A-90/09451**

- **DATABASE WPIL Section Ch, Week 8651, Derwent Publications Ltd., London, GB; Class D13, AN 86-336016**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 507 323 B1

## Description

This invention relates to a process for preparing fatty acid esters of saccharides which is adapted for industrial production and wherein fatty acid monoesters of saccharides including saccharide fatty acid monoesters and alkyl glycoside fatty acid monoesters can be prepared in an efficient manner.

Since fatty acid esters of saccharides such as glucose fatty acid esters and alkyl glucoside fatty acid esters, and particularly, their monoesters, have good characteristics on use as a surface active agent, we already proposed in European Patent Application EP-A-0 413 307 fatty acid monoesters of saccharides which are formulated in hair cosmetics along with a variety of processes for selectively preparing fatty acid monoesters of saccharides.

These processes comprise reaction between saccharides and fatty acids or lower alcohol esters thereof in organic solvents by the use of hydrolases such as a lipase. The above proposed processes are advantageous over known preparation processes of fatty acid esters of saccharides in the selective preparation of the monoesters. However, in order to attain a conversion ratio of not less than 90%, a reaction time of about 24 hours is usually necessary.

This has to be shortened in view of the industrial production efficiency. Accordingly, there is a demand of completing the reaction within a shorter time.

Aside from the processes proposed by us, there have been further proposed other processes for preparing saccharide fatty acid esters by reaction between saccharides and fatty acids in the presence of organic solvents by the use of hydrolases as set out in Japanese Laid-open Patent Application No. 61-268192 and WO89/01480 and WO090/09451. These processes have the problem that selectivity in the production of monoesters is poor, coupled with another problem that it takes about 24 hours before completion of the reaction as in the prior art processes stated hereinabove.

It is accordingly an object of the invention to provide a process for preparing fatty acid monoesters of saccharides wherein the selectivity in the preparation of the monoesters is high.

It is another object of the invention to provide a process for preparing fatty acid monoesters of saccharides wherein the reaction is completed within a short time with a good production efficiency and which process is thus adapted for industrial production.

In order to attain the above objects, we made intensive studies and, as a result, found that for the reaction between at least one saccharide selected from monosaccharides having from 5 to 7 carbon atoms, disaccharides consisting of hexoses, sugar alcohols having from 4 to 6 carbon atoms, ether compounds consisting of monosaccharides having from 5 to 7 carbon atoms and monohydric alcohols, and ether compounds consisting of hexose disaccharides and monohydric alcohols and at least one member selected from saturated and unsaturated fatty acids having from 6 to 22 carbon atoms and esters of the above-defined fatty acids and lower alcohols in the presence of organic solvents by the use of a hydrolase such as thermostable lipase, it is effective from the standpoint of the selectivity to the monoesters and the shorter reaction time to use, as the organic solvent, at least one member selected from the group consisting of acetylacetone, ethylene carbonate, propylene carbonate, γ-butyrolactone and heterocyclic compounds of the following general formula (1)

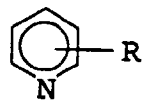

(1)

wherein R represents a linear or branched alkyl or alkenyl group having from 1 to 3 carbon atoms.

In our proposed processes set out hereinbefore, there are used secondary and tertiary alcohols as the organic solvent. The use of these alcohols along with a thermostable immobilized enzyme such as thermostable immobilized lipase ensures selective preparation of saccharide fatty acid monoesters. However, it may take about 24 hours before completion of the reaction as set forth before.

We found that when at least one member selected from acetylacetone, ethylene carbonate, propylene carbonate, γ-butyrolactone and heterocyclic compounds of the afore-indicated general formula (1) is used, the conversion becomes high with a high ratio of formation of monoesters. The reaction between saccharides and fatty acids can be accomplished within about 10 hours at a conversion of not less than about 90%. Especially, when the enzyme concentration is increased, the reaction is completed within about 2 hours without increasing the amount of diesters. This is very advantageous from the industrial viewpoint. In addition, the reaction proceeds smoothly even when the solvent is used in small amounts. Accordingly, the reaction may be performed at high substrate concentrations. This will lead to the fact that the capacity of a reaction tank required for reaction of substrates in given amounts can be made small, resulting in reduced costs of production apparatus. Moreover, unlike the solvent such as diacetone alcohol, the solvent defined above undergoes little thermal decomposition, so that after completion of the reaction, it can be collected from the

reaction system at a high rate and can be repeatedly used and collected for the reaction at temperatures of not lower than 70°C. During the course of the reaction, where water and lower alcohols which are by-produced in the reaction system are removed, a simple reduced pressure technique is sufficient to remove and collect the water and lower alcohols alone since the solvent is not entrained with the water and lower alcohols. In this regard, the process is industrially advantageous.

In addition, the use of the solvent does not lead to a lowering of the selectivity to the monoester but while suppressing the by-production of multi-substituted products such as diesters, the monoester can be selectively obtained. Therefore, the use of the solvent results in production of fatty acid monoesters of saccharides in high selectivity and in a very efficient and economical manner and ensures high enzymatic activity.

In the preparation of the fatty acid esters, it is desirable to repeatedly use expensive enzymes in order to efficiently and beneficially obtain fatty acid esters of saccharides. Hence, there is a demand of keeping the activity of the enzyme over a long term. To meet the demand, we found that when at least one member selected from styrene-based chelate resins, styrene-based, weakly basic, anionic exchange resins, and acrylic, weakly basic, anionic exchange resins is added, the activity of the enzyme can be kept over a long term and the enzyme can be repeatedly used several times.

According to the invention, there is provided a process for preparing fatty acid esters of saccharides which comprises subjecting at least one saccharide selected from monosaccharides having from 5 to 7 carbon atoms, disaccharides consisting of hexoses, sugar alcohols having from 4 to 6 carbon atoms, ether compounds consisting of monosaccharides having from 5 to 7 carbon atoms and monohydric alcohols, and ether compounds consisting of hexose disaccharides and monohydric alcohols, to reaction with at least one member selected from saturated and unsaturated fatty acids having from 6 to 22 carbon atoms and esters of the above-defined fatty acids and lower alcohols in the presence of an organic solvent by the use of a hydrolase, the improvement characterized in that the organic solvent used is at least one member selected from the group consisting of acetylacetone, ethylene carbonate, propylene carbonate, γ-butyrolactone and heterocyclic compounds of the following general formula (1)

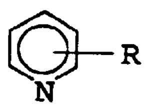

(1)

wherein R represents a linear or branched alkyl or alkenyl group having from 1 to 3 carbon atoms.

By the process, the reaction proceeds at a high conversion and is completed within a short time, thereby selectively obtaining fatty acid monoesters of saccharides. In addition, the amount of the solvent for the reaction can be reduced, which is favorable from the industrial standpoint.

Preferably, at least one selected from styrene-based, chelate resins, styrene-based, weakly basic, anionic exchange resins, and acrylic, weakly basic, anionic exchange resins are added to the reaction system. By this, the activity of the enzyme can be kept over a long term and the expensive enzyme can be repeatedly used.

The sole figure is a graph showing the relation between the conversion ratio into methyl glucoside caprylate or the content of a corresponding diester and the reaction time for different preparation processes of Example 1 and Comparative Example 1.

In the process for preparing fatty acid esters of saccharides according to the present invention, the first starting material is saccharides. The saccharides used in the present invention include monosaccharides having from 5 to 7 carbon atoms, a hexose disaccharide, sugar alcohols having from 4 to 6 carbon atoms, ether compounds consisting of monosaccharides having from 5 to 7 carbon atoms and monohydric alcohols, and ether compounds consisting of a hexose disaccharide and monohydric alcohols.

The monosaccharides having five carbon atoms include arabinose, ribose, xylose, lyxose, xylulose, ribulose, 2-deoxyribose and the like. The monosaccharides having six carbon atoms include glucose, galactose, fructose, mannose, sorbose, talose, 2-deoxyglucose, 6-deoxygalactose, 6-deoxymannose, 2-deoxygalactose and the like. The monosaccharides having seven carbon atoms include alloheptulose, sedoheptulose, mannoheptulose, glucoheptulose and the like.

The disaccharides consisting of hexoses include maltose, sucrose, sophorose and the like.

Examples of sugar alcohols include erithritol, ribitol, xylitol, allitol, sorbitol, mannitol, garactitol and the like.

The ether compounds of monosaccharides having from 5 to 7 carbon atoms or hexose disaccharides and monohydric alcohols include those compounds which have monohydric alcohols having from 1 to 12 carbon atoms, preferably from 1 to 6 carbon atoms. The monohydric alcohols may be linear or branched, saturated or unsaturated, or substituted or unsubstituted and should preferably be methyl alcohol, ethyl alcohol, propyl alcohol and butyl alcohol.

Any limitation is not placed on the position of the ether bond between the saccharides and the monohydric alcohols. Specific examples include glycosides such as methyl glucoside, ethyl glucoside, propyl glucoside, methyl fructoside,

methyl mannoside, methyl maltoside, methyl lactoside and the like, and sugar ethers such as 6-O-methyl glucose, 6-O-methyl fructose and the like. The alkyl glycosides are those which have, as an aglycone, an alkyl group joined to the hemi-acetal (anomeric) hydroxyl group of the saccharides. The steric positions of the hemi-acetal (anomeric) hydroxyl groups after the alkyl substitution may be those of the α position alone, the β position alone and the α and β-mixed positions.

Of the saccharides, there are preferably used glucose, fructose, galactose, mannose, maltose, sucrose, sorbitol, methyl glucoside, ethyl glucoside, propyl glucoside, butyl glucoside, methyl fructoside, methyl polyglucosides such as maltoside and iso-maltoside.

In the practice of the invention, at least one saccharide selected from unsubstituted monosaccharides having from 5 to 7 carbon atoms, hexose disaccharides and sugar alcohols having from 4 to 6 carbon atoms and at least one member selected from ether compounds of the saccharides and the monohydric alcohols may be used in combination. Depending on the mixing ratio, mixtures of saccharide fatty acid esters and saccharide ether fatty acid esters can be simultaneously prepared in a high efficiency.

The second starting material used in the present invention includes fatty acids having from 6 to 22 carbon atoms or their lower alkyl esters.

The fatty acids useful in the present invention are saturated or unsaturated, linear or branched fatty acids having from 6 to 22 carbon atoms. These fatty acids may be unsubstituted or substituted with a hydroxyl group, a carbonyl group, a phenyl group or the like. Specific examples of the fatty acids include caproic acid, sorbic acid, caprylic acid, capric acid, lauric acid, myristic acid, palmitoleic acid, palmitic acid, stearic acid, iso-stearic acid, oleic acid, linolic acid, linoleic acid, pentadecanoic acid, eicosanic acid, docosanoic acid, docosenoic acid, arachidonic acid, licinoleic acid, dihydroxystearic acid and the like.

The fatty acid esters are obtained from fatty acids having from 6 to 22 carbon atoms and lower alcohols having from 1 to 6 carbon atoms, preferably from 1 to 3 carbon atoms such as methanol, ethanol, propanol and the like. Examples include methyl caproate, ethyl caproate, methyl caprate, ethyl caprate, methyl laurate, ethyl laurate, propyl laurate, methyl myristate, ethyl myristate, propyl myristate, methyl palmitate, ethyl palmitate, propyl palmitate, methyl stearate, ethyl stearate, propyl stearate, methyl oleate, ethyl oleate, propyl oleate, methyl linoleate, ethyl linoleate, propyl linoleate, methyl eicosanoate, methyl arachidonate, methyl docosanoate, methyl docosenoate and the like.

The amounts of both starting materials are appropriately controlled. In general, the fatty acids are used in an amount of from 0.9 to 20 moles, preferably from 1 to 10 moles and more preferably from 2 to 5 moles, per mole of the saccharide. If the molar ratio of the fatty acid is increased, the reaction velocity increases. However, the reaction velocity does not increase when the amount exceeds 20 moles. In view of the economy, the amount of the fatty acid should favorably be not larger than 20 moles. In the present invention, when the fatty acid is used in excess with respect to the saccharide, monoesters are predominantly produced while suppressing by-production of multi-substituted products such as diesters at a very low level.

In the process of the invention, the starting materials are reacted in a specific type of organic solvent described hereinafter by the use of a hydrolase. The hydrolases used in this reaction include, for example, lipases and/or esterases including hog milt lipases, yeast lipases belonging to the genus Candida, mold lipases belonging to the genuses Aspergillus and Mucor, bacterial lipases belonging to the genus Pseudomonas (and so on), and proteases such as, trypsin, chymotrypsin, subtilisin and the like. Thermostable lipases produced from hosts in which DNA of the above bacteria is integrated are also suitable. Preferably, those hydrolases which have a good thermostability and exhibit a maximum hydrolytic activity at a pH ranging from 5 to 10, preferably from 5.5 to 9.5, are used.

For instance, thermostable hydrolases are those which have such a heat resistance that when 50 mg of an enzyme powder is dissolved in 0.4 ml of a phosphate buffer (0.1 M, pH 7) and heated at 70°C for 30 minutes, they have a remaining activity of not less than 40%, preferably not less than 80% and more preferably not less than 95%. Specific examples include thermostable lipase derived from Candida antarctica, thermostable lipase derived from Candida tsukubaensis (ATCC 24555), thermostable lipase derived from Candida auriculariae (ATCC 24121), thermostable lipase derived from Candida humicola (ATCC 14438), thermostable lipase derived from Candida foliarum (ATCC 18820), and other lipases set out in WO88/02775 or Japanese Laid-open Patent Application No. 1-501120, and thermostable lipase derived from Mucor miehei. Examples of the thermostable proteases include one derived from Bacillus thermobroteoliquixus (Thermolysin, commercial name) and one derived from Thermus aquaticus YT-G (Aqualysin, commercial name) although not limited to those indicated above.

These hydrolases may be either a pure product or a crude product. In addition, dried products of bacteria (treated bacteria or resting bacteria) capable of producing hydrolases may also be used.

The hydrolase may be used as immobilized and the immobilizing technique may be a carrier binding technique, a crosslinkage technique or an inclusion technique. Preferably, the carrier binding technique is employed.

In the case, the immobilizing carriers include, for example, inorganic substances such as active carbon, porous glass, acid clay, silica gel, bentonite, hydroxyapatite, calcium phosphate, metal oxides and the like, natural high molecular weight compounds such as starch, gluten and the like, and synthetic polymers such as polyethylene, polypropylene, phenol-formalin resins, acrylic resins, anionic exchange resins, cationic exchange resins and the like. In the practice of the invention, there are most preferably used synthetic polymers having a porous physical form, such as, for example, porous polyethylene, porous polypropylene, porous phenol-formalin resins, and porous acrylic resins. Aside from those set out above, other immobilizing carriers may be, of course, used provided that the development of the activity of the enzyme is not impeded therewith.

The amount of the hydrolase to be immobilized on the carrier is in the range of from 0.1 to 500 mg of a protein per unit gram of the immobilizing carrier. The protein which contains 2 to 50% of the hydrolase therein should preferably be immobilized.

In the practice of the invention, the amount of the hydrolase is not critical and is preferably in the range of from 0.2 to 1 part by weight, more preferably from 0.05 to 0.8 parts by weight, and most preferably from 0.08 to 0.6 parts by weight, per part of the saccharide. If the enzyme is smaller in amount, the reaction velocity tends to be decreased. On the other hand, when the amount of the enzyme is in excess, there is a tendency toward an increasing ratio of by-production of diesters or polyesters.

As stated hereinbefore, the enzymatic reaction between the saccharide and the fatty acid or its lower alkyl ester is effected in a solvent which is made of at least one member selected from acetylacetone, ethylene carbonate, propylene carbonate, γ-butyrolactone and heterocyclic compounds of the general formula (1)

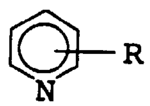

(1)

wherein R represents a linear or branched alkyl or alkenyl group having from 1 to 3 carbon atoms.

Examples of the heterocylic compounds of the formula (1) include α-picoline, β-picoline, γ-picoline, 2-ethylpyridine, 3-ethylpyridine, 4-ethylpyridine, 2-vinylpyridine, 4-vinylpyridien, 2-isopropylpyridine and the like. These organic solvents may be used singly or in combination. These organic solvents should preferably be substantially free of water.

The amount of the organic solvent depends on the type of organic solvent, the length of the carbon chain of the fatty acids or their esters and the reaction temperature. The amount should preferably be from one to ten times, more preferably from two to six times, that of the saccharide on the weight basis. Although the organic solvent may be used in larger amounts, it is preferred as stated above that the amount is not larger than 10 times the amount of the saccharide on the weight basis in view of the reaction velocity, the compactness of a reaction apparatus with a reduction of installation costs, the reduction in running costs accompanied by the compactness of the reaction apparatus and the avoidance of the cost increase in industrial production. According to the present invention, even if the organic solvent as defined hereinabove is used in such a small amount as set out above, the monoester can be predominantly, efficiently prepared at high conversion ratio and high reaction velocity while suppressing by-production of polyesters such as diesters.

The reaction system should preferably be incorporated with at least one member selected from styrene-based chelate resins, styrene-based, weakly basic, anionic exchange resins, and acrylic, weakly basic, anionic exchange resins.

Preferable resins include those which have the structures in the following formulas (1) to (5).

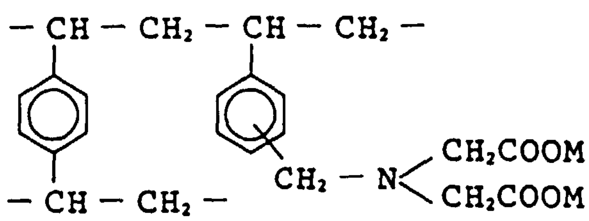

....(1)

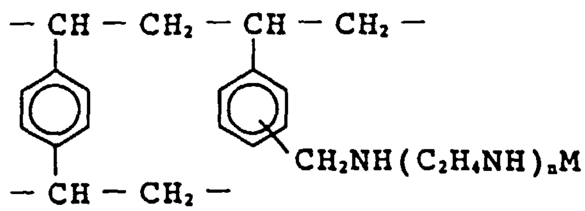

....(2)

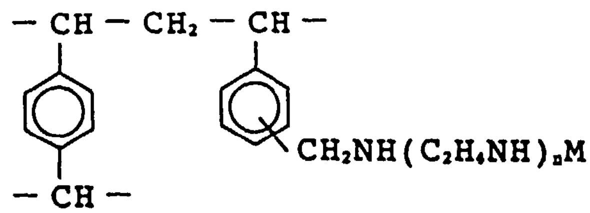

....(3)

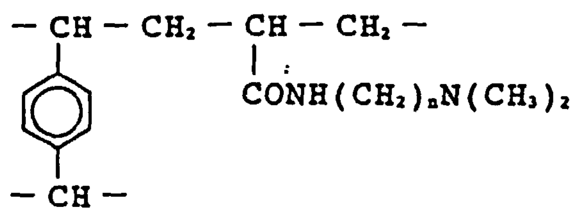

....(4)

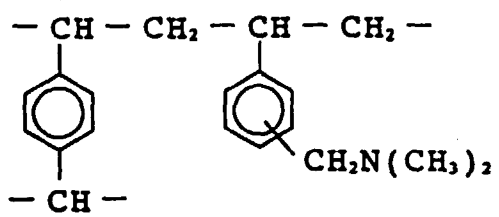

....(5)

wherein M's represent a hydrogen atom or a metal atom such as Na, K, Mg and Ca, and n is an integer of 1 to 100.

Examples of the resins which are commercially available are Diaion CR-10, CR-20, WA-20, WA-21, WA-10, WA-11 and WA-30 available from Mitsubishi Chem. Co., Ltd., and Amberlite IRC-718, IRA-68, IRA-35, IRA-93ZU, IRA-94S and Amberlist A-21 available from Organo Co., Ltd.

The amount of the resin is preferably in the range of from 0.1 to 3 times, more preferably from 0.5 to 2 times, that of the immobilized hydrolase on the weight basis. If the amount is less than 0.1 time on the weight basis, the effect of the addition of the resin is not shown. Over 3 times on the weight basis, the reaction velocity may lower in some case.

The conditions for the enzymatic reaction between the saccharide and the fatty acid or its lower alkyl ester by the use of a hydrolase may be appropriately controlled. The reaction may proceed at low temperatures. More particularly, in order to increase the reaction velocity, it is preferred that the reaction is effected at temperatures of not lower than 40°C, preferably from 50 to 100°C, and more preferably from 60 to 90°C. When the reaction is carried out under these temperature conditions, it is completed within a short time of from 1 to 10 hours, preferably from 1 to 7 hours at a conversion as high as not less than 90%. Even though the reaction is performed at such a high temperature, the enzyme is not deactivated because of the use of a thermostable hydrolase. In addition, the solvent used is stable at a high temperature ranging from 70 to 200°C, so that the productivity can be maintained over a long term.

For the preparation of saccharide fatty acid monoesters or alkyl glycoside fatty acid monoesters according to the process of the invention, there are used a batchwise procedure wherein a substrate solution and a hydrolase are introduced into a reaction vessel, agitated and shaken for the reaction and a continuous agitated vessel procedure wherein the reaction of the batchwise procedure is continuously performed.

Since the reaction of the present invention is carried out without inactivation of enzymes, the long-term continuous reaction or the repeated batchwise reaction can be performed without any trouble, thus being very advantageous in an industrial sense.

In the process of the invention, water or lower alcohols are by-produced by the enzymatic reaction. The reaction should preferably be carried out efficiently while removing the by-products from the system in such a way that the concentration of the side products in the system is not larger than 0.5 wt%, preferably not larger than 0.1 wt%. For the removal, there are used a method wherein the by-product is removed by adsorption on zeolite, molecular sieves or Glauber's salt in and/or outside the reaction system, a method wherein dried air or an inert gas is introduced into the reaction vessel for evaporation into the gas and a method wherein the reaction vessel is vacuumized and the by-product is evaporated and exhausted outside the reaction vessel. When using the enzymatic reaction apparatus in combination with these removal methods, the reaction can be efficiently carried out.

It will be noted that the solvents used in the process of the invention have, respectively, a boiling point as follows: 141°C/760 Torr for acetylacetone, 243-4°C/740 Torr for ethylene carbonate, 240°C/760 Torr for propylene carbonate, and 204-5°C/760 Torr. for γ-butyrolactone. butyrolactone. The boiling points of these solvents are significantly different from those of water and lower alcohols as will be by-produced. The heterocyclic compounds have also boiling points which are significantly higher than the boiling points of water and lower alcohols by-produced during the course of the reaction. Accordingly, the gas introducing method or the reduced pressure method for which a simple apparatus is used with little burden on apparatus costs may be adopted. For instance, if an appropriate reflux condenser for partial condensation is provided, by-produced water or lower alcohols alone can be collected at high concentrations without entrainment of the solvent. Thus, the process of the invention can bring about the reduction of production costs since the collected lower alcohols are re-utilized with ease.

Using a partial condenser, the degree of vacuum, the temperature of the coolant in the partial condenser and the coolant temperature in a cold trap are selected depending on the vapor pressure of a reaction solvent at the reaction temperature and the vapor pressure of the by-produced water or lower alcohols. From the standpoint of the reaction velocity, the degree of vacuum during the reaction should be as high as possible on the condition that the reaction solvent alone is refluxed by proper control of the temperature of the coolant. The degree of vacuum is appropriately selected depending on the type of solvent and other reaction conditions and is generally in the range of not higher than 200 Torr. For instance, when γ-butyrolactone is used as the solvent, methyl caprylate is used as the fatty acid lower alkyl ester and the reaction is effected at 80°C, the degree of vacuum is preferably not higher than 20 Torr., more preferably not higher than 15 Torr., and most preferably not higher than 12 Torr. Likewise, when β-picoline is used as the solvent, methyl caprylate is used as the fatty acid lower alkyl ester and the reaction is effected at 80°C, the degree of vacuum is preferably not higher than 100 Torr, more preferably not higher than 80 Torr , and most preferably not higher than 60 Torr

The reaction mixture obtained in this manner may be purified by a usual manner. Unreacted fatty acids or lower alkyl esters thereof may be separated and collected from the reaction mixture for re-use.

In the process of the present invention, the conversion ratio is usually 90 to 100%, particularly 95 to 99% at the above reaction temperature and reaction time ranges, and the resulting product (fatty acid ester of saccharide) usually contain 95 to 100% by weight of monoester, 0 to 5% by weight of diester and the balance of triester and higher polyesters, particularly 97 to 100% by weight of monoester, 0 to 3% by diester and the balance of triester and higher polyesters.

The thus obtained saccharide fatty acid monoesters and alkyl glycoside fatty acid monoesters have good surface activity and can be effectively used in various fields. It will be noted that when monosaccharides having from 5 to 7 carbon atoms, disaccharides consisting of hexoses and sugar alcohols having from 4 to 6 carbon atoms are used as the starting material, there are obtained fatty acid monoesters of these saccharides and that when ether compounds of monosaccharides having from 5 to 7 carbon atoms and monohydric alcohols and ether compounds of disaccharides consisting of hexoses and monohydric alcohols are used as the starting material, there are obtained alkyl glycoside fatty acid monoesters.

The present invention is more particularly described by way of examples, which should not be construed as limiting the invention. Comparative examples are also shown.

Example 1

Methyl glucoside, caprylic acid, γ-butyrolactone and a neutral thermostable lipase derived from Candida antarctica and immobilized with an acrylic resin (immobilized lipase, available from NOVO Co., Ltd.) were charged into a four-necked round bottom flask equipped with an agitator, a thermometer, a mercury manometer and a reflux condenser in amounts indicated in Table 1. A dry ice/acetone trap and an aspirater were connected to the outlet of the reflux condenser, followed by reaction under reduced pressure at 80°C. The cooling water temperature of the reflux condenser was determined at 30°C, while taking a degree of vacuum into consideration, so that the γ-butyrolactone solvent alone was refluxed and methanol by-produced during the reaction and a small amount of water contained in the starting materials were collected by the trap.

After commencement of the reaction, the reaction mixture was sampled at appropriate times, at which the resultant

product was acetylated by a usual manner and subjected to gas chromatography to determine an amount of methyl glucoside caprylates (monoester and diester) and conversions.

The results are shown in Table 1 and in Fig. 1.

Examples 2 to 28

The general procedure of Example 1 was repeated using starting materials and reaction conditions indicated in Table 1 or 2 and also using neutral thermostable lipase derived from Mucor miehei (Lipozyme, available from NOVO Co., Ltd.) only in Example 5.

The results are shown in Tables 1 and 2.

Comparative Example 1

The general procedure of Example 1 was repeated except that n-decane was used as the reaction solvent. The results are shown in Table 2 and in Fig. 1.

Comparative Example 2

The general procedure of Example 1 was repeated except that starting materials and reaction conditions indicated in Table 2 were used. The results are shown in Table 2.

Table 1

| Ex. No. | Starting Materials Used | | | Fatty Acid or Ester/Saccharide (molar ratio) | Solvent/Saccharide (weight ratio) | Enzyme/Saccharide (weight ratio) | Reaction Tem. (°C) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 1 | MG | caprylic acid | γ-butyrolactone | 5 | 5 | 0.1 | 80 | 8 | 99.0 | 0.64 |
| 2 | MG | methyl caprylate | ethylene carbonate | 5 | 5 | 0.1 | 80 | 10 | 96.5 | 1.0 |
| 3 | MG | methyl caprylate | propylene carbonate | 5 | 5 | 0.1 | 80 | 9 | 96.8 | 1.4 |
| 4 | MG | methyl caprylate | cetyl-acetone | 4 | 4 | 0.15 | 90 | 10 | 96.3 | 2.7 |
| 5 | MG | M-1 | γ-butyrolactone | 4 | 4 | 0.2 | 60 | 10 | 94.1 | 1.7 |
| 6 | BG | M-1 | γ-butyrolactone | 20 | 3 | 0.1 | 85 | 10 | 95.8 | 1.2 |
| 7 | EG | M-2 | ethylene carbonate | 10 | 5 | 0.2 | 90 | 9 | 95.4 | 1.0 |
| 8 | MG | M-3 | ethylene carbonate | 5 | 5 | 0.2 | 90 | 6 | 96.0 | 0.95 |

Table 1 (Contd.)

| Ex. No. | Starting Materials Used | | | Fatty Acid or Ester/ Saccha-ride (molar ratio) | Solvent/ Saccha-ride (weight ratio) | Enzyme/ Saccha-ride (weight ratio) | Reaction Tem. (℃) | Reaction Time (Hr.) | Conver-sion (%) | Content of Diester *1 (%) |
|---|---|---|---|---|---|---|---|---|---|---|
| | Saccha-ride | Fatty Acid or Ester | Solvent | | | | | | | |
| 9 | MG | caprylic acid | γ-butyro-lactone | 5 | 5 | 0.5 | 80 | 2 | 95.7 | 1.3 |
| 10 | MG | M-4 | γ-butyro-lactone | 5 | 5 | 0.1 | 90 | 8 | 95.0 | 0.41 |
| 11 | MG | M-4 | γ-butyro-lactone | 5 | 5 | 0.1 | 90 | 10 | 98.0 | 0.50 |
| 12 | MG | M-4 | γ-butyro-lactone | 4 | 4 | 0.15 | 70 | 10 | 94.2 | 1.5 |
| 13 | MG | M-4 | γ-butyro-lactone | 5 | 5 | 0.2 | 80 | 5 | 96.4 | 0.92 |
| 14 | MG | M-4 | γ-butyro-lactone | 5 | 5 | 0.5 | 80 | 2.5 | 97.2 | 1.0 |
| 15 | MG | M-4 | γ-butyro-lactone | 5 | 5 | 0.3 | 80 | 3 | 97.8 | 1.1 |
| 16 | MG | M-4 | γ-butyro-lactone | 5 | 5 | 0.2 | 90 | 7 | 95.7 | 0.74 |

Table 2

| Ex. No. | Starting Materials Used | | | Fatty Acid or Ester/Saccharide (molar ratio) | Solvent/Saccharide (weight ratio) | Enzyme/Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 17 | MG | M-4 | γ-butyrolactone | 3 | 5 | 0.1 | 80 | 10 | 97.6 | 0.95 |
| 18 | MG | M-4 | γ-butyrolactone | 4 | 5 | 0.1 | 80 | 5 | 90.8 | 0.28 |
| 19 | MG | M-4 | γ-butyrolactone | 4 | 5 | 0.1 | 80 | 7 | 97.3 | 0.57 |
| 20 | MG | M-4 | γ-butyrolactone | 4 | 5 | 0.1 | 80 | 10 | 100 | 1.0 |
| 12 | MG | M-4 | γ-butyrolactone | 6 | 5 | 0.1 | 80 | 7 | 99.2 | 0.98 |
| 22 | MG | M-4 | γ-butyrolactone | 10 | 5 | 0.1 | 80 | 4 | 96.2 | 1.1 |
| 23 | MG | M-4 | γ-butyrolactone | 5 | 4 | 0.1 | 80 | 5 | 99.1 | 0.96 |
| 24 | MG | M-4 | γ-butyrolactone | 5 | 3 | 0.1 | 80 | 5 | 96.8 | 1.3 |

Table 2 (Contd.)

| Ex. No. | Starting Materials Used | | | Fatty Acid or Ester/ Saccharide (molar ratio) | Solvent/ Saccharide (weight ratio) | Enzyme/ Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 25 | MG | M − 4 | γ − butyrolactone | 4 | 4 | 0.1 | 80 | 5 | 98.2 | 0.73 |
| 26 | MG | M − 4 | γ − butyrolactone | 3 | 4 | 0.1 | 80 | 10 | 95.8 | 0.99 |
| 27 | MG | ethyl caprylate | γ − butyrolactone | 10 | 5 | 0.1 | 80 | 8 | 95.3 | 1.0 |
| 28 | MG | isopropyl caproate | γ − butyrolactone | 10 | 5 | 0.1 | 80 | 10 | 95.0 | 1.1 |
| Comparative Example | | | | | | | | | | |
| 1 | MG | caprylic acid | n − decane | 5 | 5 | 0.1 | 80 | 8<br>15 | 34.8<br>62.2 | 12.7<br>23.5 |
| 2 | MG | caprylic acid | diacetone alcohol | 5 | 5 | 0.1 | 40 | 8 | 38.9 | trace |

In the above Tables, the marks or symbols used have the following meanings.

MG: methyl glucoside
BG: butyl glucoside
EG: ethyl glucoside
M-1: a mixture of 3% of methyl caproate and 97% of methyl caprylate
M-2: mixture of 85% of methyl caprate, 10% of methyl laurate and 5% of methyl myristate
M-3: a mixture of 90% of methyl palmitate, 7% of methyl oleate, 2% of methyl linoleate and 1% of methyl stearate
M-4: a mixture of 3% of methyl caproate, 50% of methyl caprylate and 47% of methyl caprate
Note*: The content of the diester was determined from the following equation:

$$\frac{\text{Fatty acid diester of saccharide}}{\text{Fatty acid mono and diesters of saccharide}} \times 100$$

Examples 29 to 36

The general procedure of Example 1 was repeated using starting materials and reaction conditions indicated in Table 3, thereby obtaining saccharide fatty acid esters and alkyl glycoside fatty acid esters. The enzyme used was such a lipase as used in Example 1.

Table 3

| Ex. No. | Starting Materials Used | | | Fatty Acid or Ester/ Saccharide (molar ratio) | Solvent/ Saccharide (weight ratio) | Enzyme/ Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 29 | glucose | methyl caprylate | γ-butyrolactone | 4 | 5 | 0.1 | 80 | 10 | 99.6 | 1.2 |
| 30 | fructose | methyl caprylate | γ-butyrolactone | 5 | 5 | 0.1 | 80 | 7 | 98.7 | 0.69 |
| 31 | xylose | methyl caprylate | γ-butyrolactone | 5 | 5 | 0.1 | 80 | 8 | 99.0 | 0.72 |
| 32 | glucoheptulose | methyl caprylate | γ-butyrolactone | 4.5 | 4.5 | 0.1 | 80 | 10 | 97.0 | 1.3 |
| 33 | sucrose | methyl caprylate | γ-butyrolactone | 4 | 6 | 0.15 | 80 | 10 | 98.9 | 1.2 |
| 34 | sorbitol | methyl caprylate | γ-butyrolactone | 4 | 6 | 0.1 | 80 | 7 | 98.0 | 0.95 |
| 35 | methyl lactoside | methyl caprylate | γ-butyrolactone | 4 | 4 | 0.1 | 80 | 7 | 98.5 | 0.77 |
| 36 | propyl glucoside | methyl caprylate | γ-butyrolactone | 5 | 6 | 0.2 | 90 | 6 | 97.1 | 1.4 |

Example 37

Methyl glucoside, caprylic acid, β-picoline and a neutral thermostable lipase derived from Candida antarctica and immobilized with an acrylic resin (immobilized lipase, available from NOVO Co., Ltd.) were charged into a four-necked round bottom flask equipped with an agitator, a thermometer, a mercury manometer and a reflux condenser in amounts indicated in Table 4. A dry ice/acetone trap and a water jet pump were connected to the outlet of the reflux condenser, followed by reaction under reduced pressure at 80°C. The cooling water temperature of the reflux condenser was determined at 40°C, while taking a degree of vacuum into consideration, so that the β-picoline solvent alone was refluxed and methanol by-produced during the reaction and a small amount of water contained in the starting materials were collected by the trap.

After commencement of the reaction, the reaction mixture was sampled at appropriate times, at which the resultant product was acetylated by a usual manner and subjected to gas chromatography to determine an amount of methyl glucoside caprylates (monoester and diester) and conversions. The results are shown in Table 4.

Examples 38 to 66

The general procedure of Examples 37 was repeated using starting materials and reaction conditions indicated in Table 4 or 5, thereby obtaining alkyl glycoside/fatty acid esters.

With regard to the enzyme, the same lipase as used in Example 37 was used except that in Example 43, there was used a neutral, thermostable lipase (Lipozyme, available from NOVO Co., Ltd.) derived from Mucor miehei. The results are shown in Tables 4 and 5.

Table 4

| Ex. No. | Starting Materials Used | | | Fatty Acid or Ester/ Saccharide (molar ratio) | Solvent/ Saccharide (weight ratio) | Enzyme/ Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 37 | MG | caprylic acid | α-picoline | 5 | 5 | 0.1 | 80 | 3.5 | 95.3 | 0.61 |
| 38 | MG | methyl caprylate | β-picoline | 5 | 5 | 0.1 | 80 | 4 | 98.4 | 1.1 |
| 39 | MG | methyl caprylate | γ-picoline | 5 | 5 | 0.1 | 80 | 4 | 97.6 | 0.76 |
| 40 | MG | methyl caprylate | 2-ethyl-pyridine | 4 | 4 | 0.15 | 90 | 6 | 95.9 | 1.6 |
| 41 | MG | methyl caprylate | 2-propyl-pyridine | 5 | 5 | 0.1 | 80 | 6 | 97.0 | 2.1 |
| 42 | MG | methyl caprylate | 2-vinyl-pyridine | 5 | 5 | 0.1 | 80 | 7 | 95.1 | 1.8 |
| 43 | MG | M-1 | β-picoline | 4 | 4 | 0.2 | 60 | 3.5 | 94.5 | 1.7 |
| 44 | BG | M-1 | β-picoline | 20 | 3 | 0.1 | 85 | 3.5 | 95.7 | 1.4 |
| 45 | BG | M-2 | α-picoline | 10 | 5 | 0.2 | 90 | 4 | 96.0 | 1.3 |

Table 4 (Contd.)

| Ex. No. | Starting Materials Used | | | Fatty Acid or Ester/ Saccharide (molar ratio) | Solvent/ Saccharide (weight ratio) | Enzyme/ Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 46 | MG | M − 3 | α-picoline | 5 | 5 | 0.2 | 90 | 3 | 97.3 | 1.1 |
| 47 | MG | caprylic acid | β-picoline | 5 | 5 | 0.5 | 80 | 1 | 97.4 | 1.5 |
| 48 | MG | M − 4 | β-picoline | 5 | 5 | 0.1 | 90 | 3 | 95.9 | 0.58 |
| 49 | MG | M − 4 | β-picoline | 5 | 5 | 0.1 | 90 | 3.5 | 99.0 | 1.1 |
| 50 | MG | M − 4 | β-picoline | 4 | 4 | 0.15 | 70 | 4 | 95.6 | 1.6 |
| 51 | MG | M − 4 | β-picoline | 5 | 5 | 0.2 | 80 | 2.5 | 96.1 | 1.4 |
| 52 | MG | M − 4 | β-picoline | 5 | 5 | 0.5 | 80 | 1.5 | 98.6 | 1.6 |
| 53 | MG | M − 4 | β-picoline | 5 | 5 | 0.3 | 80 | 2 | 97.8 | 1.3 |
| 54 | MG | M − 4 | β-picoline | 5 | 5 | 0.2 | 90 | 2.5 | 95.7 | 1.1 |
| 55 | MG | M − 4 | β-picoline | 3 | 5 | 0.1 | 80 | 5 | 97.3 | 0.97 |

Table 5

| Ex. No. | Starting Materials Used | | | Fatty Acid or Ester/Saccharide (molar ratio) | Solvent/Saccharide (weight ratio) | Enzyme/Saccharide (weight ratio) | Reaction Tem. (℃) | Reaction Time (Hr.) | Conversion (%) | Content of Diester *1 (%) |
|---|---|---|---|---|---|---|---|---|---|---|
| | Saccharide | Fatty Acid or Ester | Solvent | | | | | | | |
| 56 | MG | M-4 | β-picoline | 4 | 5 | 0.1 | 80 | 3 | 90.1 | 0.42 |
| 57 | MG | M-4 | β-picoline | 4 | 5 | 0.1 | 80 | 3.5 | 94.3 | 0.56 |
| 58 | MG | M-4 | β-picoline | 4 | 5 | 0.1 | 80 | 4 | 97.9 | 1.0 |
| 59 | MG | M-4 | β-picoline | 6 | 5 | 0.1 | 80 | 3 | 95.6 | 0.65 |
| 60 | MG | M-4 | β-picoline | 10 | 5 | 0.1 | 80 | 2 | 96.2 | 0.86 |
| 61 | MG | M-4 | β-picoline | 5 | 4 | 0.1 | 80 | 3 | 98.3 | 1.2 |
| 62 | MG | M-4 | β-picoline | 5 | 3 | 0.1 | 80 | 3 | 96.7 | 1.4 |
| 63 | MG | M-4 | β-picoline | 4 | 4 | 0.1 | 80 | 3 | 97.9 | 1.2 |
| 64 | MG | M-4 | β-picoline | 3 | 4 | 0.1 | 80 | 4.5 | 96.5 | 0.98 |
| 65 | MG | caprylic acid | β-picoline | 10 | 5 | 0.1 | 80 | 4 | 95.3 | 1.4 |
| 66 | MG | isopropyl caproate | β-picoline | 10 | 5 | 0.1 | 80 | 4.5 | 95.2 | 1.5 |

In the above Tables, the marks or symbols used have the following meanings.

MG: methyl glucoside
BG: butyl glucoside
EG: ethyl glucoside
M-1: a mixture of 3% of methyl caproate and 97% of methyl caprylate
M-2: a mixture of 85% of methyl caprate, 10% of methyl laurate and 5% of methyl myristate
M-3: a mixture of 90% of methyl palmitate, 7% of methyl oleate, 2% of methyl linoleate and 1% of methyl stearate
M-4: a mixture of 3% of methyl caproate, 50% of methyl caprylate and 47% of methyl caprate
Note*: The content of the diester was determined from the following equation:

$$\frac{\text{Fatty acid diester of saccharide}}{\text{Fatty acid mono and diesters of saccharide}} \times 100$$

Examples 67 to 74

The general procedure of Example 37 was repeated using starting materials and reaction conditions indicated in Table 6, thereby obtaining saccharide fatty acid esters and alkyl glycoside fatty acid esters. The enzyme used was such a lipase as used in Example 37.

Table 6

| Ex. No. | Starting Materials Used | | | Fatty Acid or Ester/ Saccha-ride (molar ratio) | Solvent/ Saccha-ride (weight ratio) | Enzyme/ Saccha-ride (weight ratio) | Reaction Tem. (℃) | Reaction Time (Hr.) | Conver-sion (%) | Content of Diester *1 (%) |
|---|---|---|---|---|---|---|---|---|---|---|
| | Saccha-ride | Fatty Acid or Ester | Solvent | | | | | | | |
| 67 | glucose | methyl caprylate | β-picoline | 4 | 5 | 0.1 | 80 | 4.5 | 98.7 | 1.5 |
| 68 | fructose | methyl caprylate | β-picoline | 5 | 5 | 0.1 | 80 | 3.5 | 97.5 | 1.0 |
| 69 | xylose | methyl caprylate | β-picoline | 5 | 5 | 0.1 | 80 | 4 | 98.4 | 1.1 |
| 70 | gluco-heptulose | methyl caprylate | β-picoline | 4.5 | 4.5 | 0.1 | 80 | 4.5 | 95.8 | 1.6 |
| 71 | sucrose | methyl caprylate | β-picoline | 4 | 6 | 0.15 | 80 | 4.5 | 96.0 | 1.4 |
| 72 | sorbitol | methyl caprylate | β-picoline | 4 | 6 | 0.1 | 80 | 3.5 | 97.7 | 1.2 |
| 73 | methyl lactoside | methyl caprylate | β-picoline | 4 | 4 | 0.1 | 80 | 3.5 | 97.0 | 1.1 |
| 74 | propyl glucoside | methyl caprylate | β-picoline | 5 | 6 | 0.2 | 90 | 3 | 96.7 | 1.7 |

Example 75

The general procedure of Example 63 was repeated using the same starting materials and apparatus as used in Example 63 but using the following reaction conditions:

Fatty acid ester/saccharide = 4 (by molar ratio)
Solvent/saccharide = 4 (ratio by weight)
Enzyme/saccharide = 0.1 (ratio by weight)

After reaction at a temperature of 80°C for predetermined times while refluxing under reduced pressure, the reaction system was returned to normal pressures and agitation was stopped. Thereafter, the system was allowed to stand at room temperature for 1 hour thereby causing the immobilized enzyme to be settled. The supernatant reaction solution alone was carefully collected while inserting a pipette to a depth of the solution just above a level at which the immobilized enzyme was suckingly taken up therein.

Then, fresh starting materials other than the enzyme were charged into the four - necked round bottom flask, in which the immobilized enzyme and a small amount of the reaction solution were left, under the following different conditions so that the enzyme was repeatedly used for the second cycle of the reaction.

The above procedure was repeated to repeatedly use the enzyme to effect the twenty cycles of the reaction in total.

The results of the reaction time, the conversion and the content of diesters after the repetitions of the reaction are shown in Table 7.

Table 7

| Number of reaction (cycles) | Reaction time (Hr.) | Conversion (%) | Content of diesters (%) |
|---|---|---|---|
| 1 | 3.0 | 98.1 | 1.2 |
| 10 | 3.5 | 97.1 | 1.1 |
| 20 | 4.0 | 98.8 | 1.3 |

Examples 75 to 79

Methyl glucoside, methyl caprylate, γ-butyrolactone and a neutral thermostable lipase derived from Candida antarctica and immobilized with an acrylic resin (immobilized lipase, available from NOVO Co., Ltd.) were charged into a four-necked round bottom flask equipped with an agitator, a thermometer, a mercury manometer and a reflux condenser in amounts indicated in Table 8. Further, resins of the formulas indicated in Table 8 were added to the mixture in amounts indicated in the table. A dry ice/acetone trap and an aspirator were connected to the outlet of the reflux condenser, followed by reaction under reduced pressure at 80°C. The cooling water temperature of the reflux condenser was determined at 30°C, while taking a degree of vacuum into consideration, so that the γ-butyrolactone solvent alone was refluxed and methanol by-produced during the reaction and a small amount of water contained in the starting materials were collected by the trap.

Fatty acid ester/saccharide = 4 (molar ratio)
Reaction solvent/saccharide = 4 (ratio by weight)
Enzyme/saccharide = 0.1 (ratio by weight)
Reaction temperature = 80°C, refluxing under reduced pressure

After the reaction for given times, the system was returned to normal pressure and the agitation was stopped, followed by allowing to stand at room temperature for 1 hour thereby causing the immobilized enzyme to be settled. Thereafter, the resultant supernatant reaction solution was carefully collected while inserting a pipette to a depth of the reaction solution just above the settled, fixed enzyme.

Fresh starting materials other than the enzyme were charged into the four-necked round bottom flask in which the fixed enzyme and a small amount of the reaction solution remained, and subjected to the second reaction by repeated use of the enzyme.

In this manner, the enzyme was repeatedly used to effect a given cycle of reactions.

The results of the repeated reactions are shown in Table 8 with respect to the reaction time, the conversion and the content of diesters. The conversion and the diester content were, respectively, determined by sampling the reaction mixture at appropriate intervals and acetylating the resultant product in the mixture by a usual manner, followed by gas chromatography to calculate the amount of methyl glucoside caprylates (monoester and diester) and the conversion.

Table 8

| Ex. No. | Type (commercial name) | Resins structure (structure set out in catalogues) | Amount resin/ fixed enzyme (ratio by weight) | Reaction cycle | Reaction tiem (Hours) | Con-version (%) | Content of diester ** (%) |
|---|---|---|---|---|---|---|---|
| 75 | Diaion CR-10 (registered trade name) | 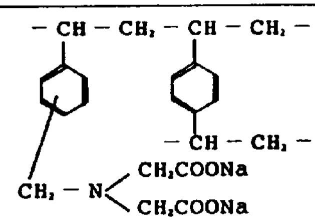 | 2 | 1 | 5 | 96.9 | 0.73 |
| | | | | 5 | 10 | 96.2 | 0.72 |
| 76 | Diaion CR-20 (registered trade name) | 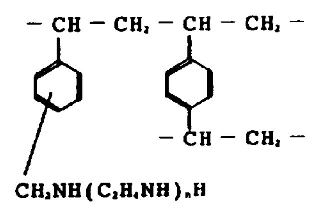 | 2 | 1 | 5 | 98.7 | 0.95 |
| | | | | 4 | 7 | 95.0 | 0.61 |
| | | | | 8 | 10 | 95.8 | 0.36 |
| 77 | Diaion WA-20 (registered trade Name) | 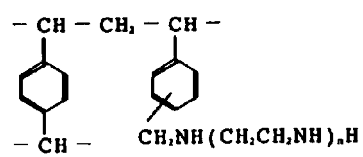 | 2 | 1 | 4 | 97.6 | 1.2 |
| | | | | 5 | 8 | 94.8 | 0.80 |

Table 8 (Contd.)

| Ex. No. | Type (commercial name) | Resins structure (structure set out in catalogues) | Amount resin/ fixed enzyme (ratio by weight) | Reaction cycle | Reaction tiem (Hours) | Con-version (%) | Content of diester ** (%) |
|---|---|---|---|---|---|---|---|
| 78 | Diaion WA-10 (registered trade name) | 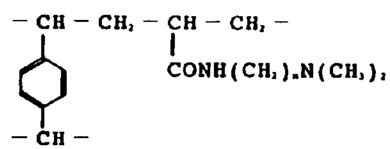 | 0.5 | 1 | 4 | 97.9 | 1.1 |
| | | | | 10 | 8 | 97.0 | 0.80 |
| | | | 2 | 1 | 5 | 98.4 | 0.90 |
| | | | | 5 | 7 | 97.5 | 1.2 |
| | | | | 10 | 8.5 | 97.7 | 0.85 |
| | | | | 15 | 9 | 97.3 | 0.81 |
| | | | 3 | 1 | 5 | 96.5 | 0.77 |
| | | | | 3 | 7 | 97.6 | 0.68 |
| 79 | Diaion WA-30 (registered trade name) | 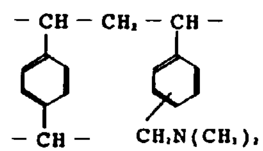 | 2 | 1 | 4 | 96.6 | 0.99 |
| | | | | 5 | 7 | 97.2 | 0.76 |
| | | | | 10 | 10 | 97.5 | 0.80 |

**Claims**

1. A process for preparing fatty acid esters of saccharides which comprises subjecting at least one saccharide selected from monosaccharides having from 5 to 7 carbon atoms, disaccharides consisting of hexoses, sugar alcohols having from 4 to 6 carbon atoms, ether compounds consisting of monosaccharides having from 5 to 7 carbon atoms and monohydric alcohols, and ether compounds consisting of hexose disaccharides and monohydric alcohols, to reaction with at least one member selected from saturated and unsaturated fatty acids having from 6 to 22 carbon atoms and esters of said fatty acids and lower alcohols in the presence of an organic solvent by the use of a hydrolase, said organic solvent being at least one member selected from the group consisting of acetylacetone, ethylene carbonate, propylene carbonate, γ-butyrolactone and heterocyclic compounds of the following general formula (1)

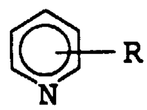 (1)

wherein R represents a linear or branched alkyl or alkenyl group having from 1 to 3 carbon atoms.

2. A process according to Claim 1, wherein the monohydric alcohol for both ether compounds has from 1 to 12 carbon atoms.

3. A process according to Claim 1 or 2 wherein at least one saccharide is glucose, fructose, galactose, mannose, maltose, sucrose, sorbitol, methyl glucoside, ethyl glucoside, propyl glucoside, butyl glucoside, methyl fructoside or a methyl polyglucoside.

4. A process according to any one of claims 1 to 3, wherein said at least one member selected from fatty acids and their lower alcohol esters is used in an amount of from 0.9 to 20 moles per mole of said saccharide.

5. A process according to any one of claims 1 to 4, wherein said hydrolase is resistant to heat and has a maximum hydrolytic activity in a pH range of from 5 to 10.

6. A process according to any one of claims 1 to 5, wherein said hydrolase is immobilized on a carrier.

7. A process according to any one of claims 1 to 6 wherein said hydrolase is immobilized in an amount of from 0.1 to 500 mg of proteins per unit gram of the carrier in such a way that said hydrolase is contained at from 2 to 50% of the proteins.

8. A process according to any one of claims 1 to 7, wherein said organic solvent is substantially free of water.

9. A process according to any one of claims 1 to 8, wherein said organic solvent is used in an amount of one to ten times that of said saccharide on the weight basis.

10. A process according to any one of claims 1 to 9, further comprising adding at least one resin selected from the group consisting of styrene-based chelate resins, styrene-based, weakly basic anionic exchange resins, and acrylic, weakly basic anionic exchange resins, to a reaction mixture of said at least one saccharide and said at least one member.

11. A process according to claim 10, wherein said at least one resin is added in an amount of from 0.1 to 3 times that of the hydrolase on the weight basis.

**Patentansprüche**

1. Verfahren zur Herstellung von Fettsäuresaccharidestern, umfassend Umsetzen wenigstens eines Saccharids, gewählt aus Monosacchariden mit 5 bis 7 Kohlenstoffatomen, Disacchariden, die aus Hexosen bestehen, Zuckeralkoholen mit 4 bis 6 Kohlenstoffatomen, Etherverbindungen, die aus Monosacchariden mit 5 bis 7 Kohlenstoffatomen und einwertigen Alkoholen bestehen, sowie Etherverbindungen, die aus Hexosedisacchariden und einwertigen Alkoholen bestehen, mit wenigstens einem Vertreter, gewählt aus gesättigten oder ungesättigten Fettsäuren mit 6 bis 22 Kohlenstoffatomen, sowie Estern der Fettsäuren mit Niederalkoholen, in Gegenwart eines organischen Lösungsmittels unter Verwendung einer Hydrolase, wobei das organische Lösungsmittel wenigstens ein Vertreter ist, gewählt aus der Gruppe, bestehend aus Acetylaceton, Ethylencarbonat, Propylencarbonat, γ-Butyrolacton sowie heterocyclischen Verbindungen der folgenden allgemeinen Formel (1),

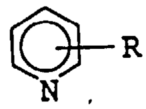 ,

(1)

wobei R einen geraden oder verzweigten Alkyl- oder Alkenylrest mit 1 bis 3 Kohlenstoffatomen darstellt.

**2.** Verfahren nach Anspruch 1, wobei der einwertige Alkohol für beide Etherverbindungen 1 bis 12 Kohlenstoffatome besitzt.

**3.** Verfahren nach Anspruch 1 oder 2, wobei wenigstens ein Saccharid Glucose, Fructose, Galactose, Mannose, Maltose, Saccharose, Sorbit, Methylglycosid, Ethylglycosid, Propylglycosid, Butylglycosid, Methylfructosid oder ein Methylpolyglycosid darstellt.

**4.** Verfahren nach einem derAnsprüche 1 bis 3, wobei wenigstens ein Vertreter, gewählt aus Fettsäuren und ihren Niederalkoholestern, in einer Menge von 0,9 bis 20 Mol je Mol Saccharid eingesetzt wird.

**5.** Verfahren nach einem der Ansprüche 1 bis 4, wobei die Hydrolase gegen Hitze beständig ist und eine maximale hydrolytische Aktivität in einem Bereich des pH-Werts von 5 bis 10 besitzt.

**6.** Verfahren nach einem der Ansprüche 1 bis 5, wobei die Hydrolase auf einem Träger immobilisiert ist.

**7.** Verfahren nach einem der Ansprüche 1 bis 6, wobei die Hydrolase in einer Menge von 0,1 bis 500 mg Protein je Gramm auf dem Träger in der Art immobilisiert wird, daß die Hydrolase in 2 bis 50 Gewichts-% der Proteine enthalten ist.

**8.** Verfahren nach einem der Ansprüche 1 bis 7, wobei das organische Lösungsmittel im wesentlichen wasserfrei ist.

**9.** Verfahren nach einem der Ansprüche 1 bis 8, wobei das organische Lösungsmittel in ein- bis zehnfacher Menge des Saccharids, bezogen auf das Gewicht, eingesetzt wird.

**10.** Verfahren nach einem der Ansprüche 1 bis 9, ferner umfassend Zugeben wenigstens eines Harzes, gewählt aus der Gruppe, bestehend aus Chelatharzen auf Styrolbasis, schwach basischen, anionischen Austauscherharzen auf Styrolbasis sowie acrylischen, schwach basischen, anionischen Austauscherharzen, zu einem Reaktionsgemisch aus dem wenigstens einem Saccharid und dem wenigstens einem Vertreter.

**11.** Verfahren nach Anspruch 10, wobei das wenigstens eine Harz in einer 0,1- bis 3-fachen Menge der Hydrolase, bezogen auf das Gewicht, zugegeben wird.

## Revendications

**1.** Procédé de préparation d'esters d'acide gras avec des saccharides, qui comprend les étapes consistant à soumettre au moins un saccharide choisi parmi les monosaccharide contenant de 5 à 7 atomes de carbone, les disacharides constitués d'hexoses, les sucre-alcools contenant de 4 à 6 atomes de carbone, les composés d'éther constitués de monosaccharides contenant de 5 à 7 atomes de carbone et les alcools monovalents, à une réaction avec au moins un membre choisi parmi les acides gras saturés et insaturés contenant de 6 à 22 atomes de carbone et les esters desdits acides gras et d'alcools inférieurs, en présence d'un solvant organique, en utilisant une hydrolase, ledit solvant organique étant au moins un membre choisi dans l'ensemble constitué d'acétylacétone, de

carbonate d'éthylène, de carbonate de propylène, de γ-butyrolactone et de composés hétérocycliques répondant à la formule générale (1) suivante

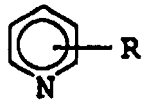

(1)

dans laquelle R représente un groupe alkyle ou alcényle linéaire ou ramifié contenant de 1 à 3 atomes de carbone.

**2.** Procédé conforme à la revendication 1, dans lequel l'alcool monovalent des deux composés d'éther contient de 1 à 12 atomes de carbone.

**3.** Procédé conforme à la revendication 1 ou à la revendication 2, dans lequel au moins un des saccharides est le glucose, le fructose, le galactose, le mannose, le maltose, le saccharose, le sorbitol, le méthylglucoside, l'éthylgucoside, le propylglucoside, le butylgucoside, le méthyflructoside ou un méthylpolyglucoside.

**4.** Procédé conforme à l'une quelconque des revendications 1 à 3, dans lequel ledit ou lesdits membres choisis parmi les acides gras et leurs esters d'alcool inférieur est utilisé en une quantité comprise entre 0,9 et 20 moles par mole dudit saccharide.

**5.** Procédé conforme à l'une quelconque des revendications 1 à 4, dans lequel ladite hydrolase est résistante à la chaleur et a une activité hydrolytique maximum dans un intervalle de pH de 5 à 10.

**6.** Procédé conforme à l'une quelconque des revendications 1 à 5, dans lequel ladite hydrolase est immobilisée sur un support.

**7.** Procédé conforme à l'une quelconque des revendications 1 à 6, dans lequel ladite hydrolase est immobilisée en une quantité comprise entre 0,1 et 500 mg de protéines par gramme du support, de telle sorte que ladite hydrolase constitue de 2 à 50 % des protéines.

**8.** Procédé conforme à l'une quelconque des revendications 1 à 7, dans lequel ledit solvant organique est pratiquement dépourvu d'eau.

**9.** Procédé selon l'une quelconque des revendications 1 à 8, dans lequel on utilise ledit solvant organique en une quantité comprise entre 1 à 10 fois la quantité dudit saccharide, en rapport pondéral.

**10.** Procédé conforme à l'une quelconque des revendications 1 à 9, comprenant en outre l'étape consistant à ajouter au moins une résine choisie dans l'ensemble constitué de résines chélatées à base de styrène, de résines d'échange d'anions faiblement basiques à base de styrène, et de résines d'échange d'anions faiblement basiques acryliques, à un mélange réactionnel dudit ou desdits saccharides et dudit ou desdits membres.

**11.** Procédé conforme à la revendication 10, dans lequel on ajoute ladite ou lesdites résines en une quantité comprise entre 0,1 et 3 fois la quantité d'hydrolase, en rapport pondéral.

FIG.1

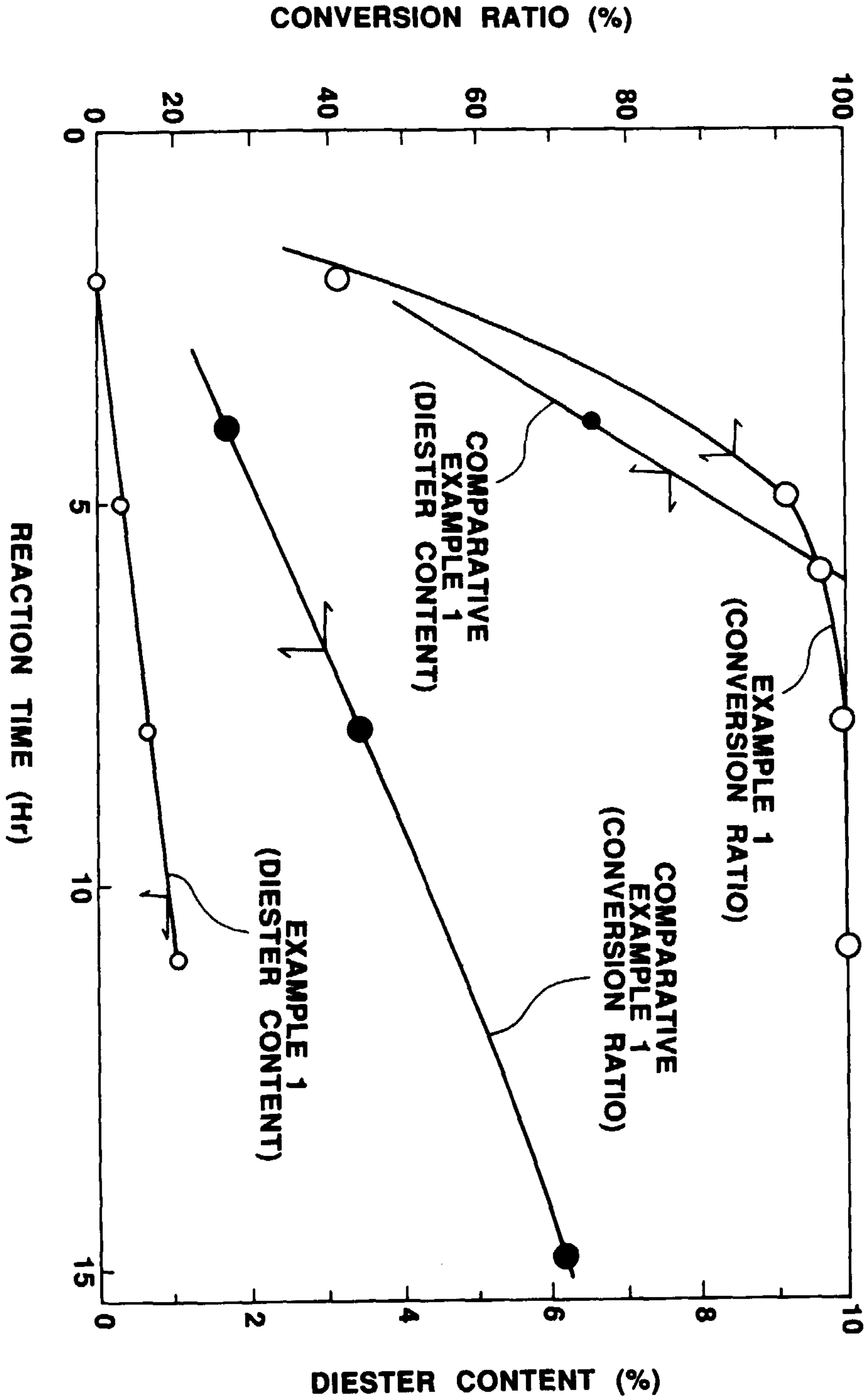
CONVERSION RATIO (%)
0
20
40
60
80
100
REACTION TIME (Hr)
0
5
10
15
DIESTER CONTENT (%)
0
2
4
6
8
10
EXAMPLE 1 (CONVERSION RATIO)
COMPARATIVE EXAMPLE 1 (DIESTER CONTENT)
COMPARATIVE EXAMPLE 1 (CONVERSION RATIO)
EXAMPLE 1 (DIESTER CONTENT)